# EUROPEAN PATENT APPLICATION

(11) **EP 4 843 975 A1**
(43) Date of publication of application: **25.11.2026**
(21) Application number: 25195922.7
(22) Date of filing: 14.08.2025
(51) Int. Cl.: C07C 17/389, C09K 13/00

(54) **DIFLUOROETHYLENE COMPOSITIONS FOR ETCH APPLICATIONS**

(30) Priority: 20.05.2025 US 202563809231 P
(71) Applicant: Arkema, Inc., Radnor PA 19087 (US); The Texas A&M University System, College Station, TX 77843-3369 (US)
(72) Inventor: Mashuga, Chad, Manvel, TX 77578-3294 (US); Kemppainen, Daniel Ralph, Chassell, MI 49916-8401 (US); Demott, Jessica Carman, Langhorne, PA 19047-8244 (US); Rodowski, Charles Damien, Downington, PA 19335-1648 (US)
(74) Representative: Bandpay & Greuter

(57) **Abstract**

The present disclosure describes compositions comprising predominantly 1,1-difluoroethylene (HFO-1132a), along with various impurities and having various characterizations such as a low GWP. Also described are methods of purifying 1,1-difluoroethylene compositions, as well as use of such purified compositions in selective etching of multilayer substrates.

## Description

### CROSS-REFERENCE TO RELATED APPLICATIONS

This application claims the benefit of U.S. Provisional Patent Application No. 63/809,231, filed on May 20, 2025, the contents of which are incorporated herein by reference.

### FIELD

The present disclosure describes compositions and methods for making difluoroethylene, which may contain some level of impurities. Also described are methods of purifying difluoroethylene compositions, as well as for using such compositions for plasma etching, *e.g.,* of multilayer substrates such as containing features spanning more than one layer.

### BACKGROUND

Strong regulatory pressure on high global warming potential etch gases has prompted the semiconductor industry, among others, to seek alternative technologies. Although there are several options, the lower global warming potential compounds are typically more difficult or involved to synthesize and/or purify. Further regulatory pressure regarding PFAS compounds, which tend to have high global warming potential, reinforces the push away from perfluorinated hydrocarbons in such etch applications.

1,1-Difluoroethylene (*aka* HFO-1132a, VDF, and/or VF2) is well known, mostly as a monomer in the formation of polymers such as poly(vinylidene fluoride), which polymer has many uses, among them as battery separator materials, as a heat transfer (refrigerant) component, and as a foam blowing agent, *inter alia.* Nevertheless, VF2 being a relatively low global warming potential compound, it has already been suggested as a potential etch gas alternative, although such suggested use has been typically as a mixture with other fluorocarbons to lower the global warming potential of the mixed composition. Examples of publications disclosing uses of VF2 are enumerated below.

U.S. Patent Application Publication No. 2025/0059115 discloses stabilization of C2 hydro-halogenated olefins, including VF2 and trifluoroethylene (VF3), using aliphatic alkenes with a boiling point not more than 80°C (*e.g.*, 1-butene). Such stabilization can be important in maintaining the monomer and avoiding oligomers, *e.g*., for polymerization purposes.

U.S. Patent Nos. 8,845,922 and 9,719,002 each disclose compositional blends useful as refrigerants, where the blend comprises VF2 and carbon dioxide or pentafluoroethane, which blend may optionally be azeotropic or azeotropic-like.

PCT Publication No. WO 2019/030508 discloses heat transfer compositions comprising HFO-1132a, HFC-32, HFO-1234yf, optionally carbon dioxide, and optionally HFO-1123.

PCT Publication No. WO 2020/214912 discloses a fluorinated alkene heat transfer composition made by contacting a fluorinated C3+ HFO with a fluorinated C2 HFO.

PCT Publication No. WO 2024/143539 discloses low-GWP refrigerant compositions comprising 99.5 mass% or more of a mixture of 1,2-difluoroethylene (HFO-1132(E)) and HFC-32, as a potential replacement for R410A.

PCT Publication No. WO 2024/213886 discloses compositions comprising HFO-1132a, HFC-32, HFO-1132(E), and one or more of HFO-1234ze, HFC-152a, and HFO-1234yf. Such compositions are disclosed as replacements for existing refrigerants such as R410A and R454B.

U.S. Patent No. 9,460,935 discloses methods of fabricating a semiconductor device with multi-stacked layers using a plasma of a gas comprising, among other combinatorial possibilities, VF2 plus a saturated fluorocarbon gas.

PCT Publication No. WO 2024/009815 discloses a substrate processing method to etch using 13-50 vol% hydrogen gas plus one or more other gases selected from VF2, HFO-1234ze, and HFO-1336mzz(E).

U.S. Patent Application Publication No. 2023/0127467 discloses plasma processing methods for etching silicon oxide-silicon nitride substrates with a first plasma using a combination of HF and a perfluorinated carbonaceous gas or an HFC gas, in tandem with an oxygen-containing gas, and a second plasma containing HF and s different perfluorinated carbonaceous gas, in tandem with an oxygen-containing gas.

U.S. Patent Application Publication Nos. 2025/0079127 and 2025/0079183 disclose selective substrate etching methods using gases comprising various isomers of C₂H₂F₂.

PCT Publication Nos. WO 2024/181008 and WO 2024/181009 disclose various methods of purification of VF2 from a composition comprising VF2 and HFC-23 by absorption and distillation, respectively.

PCT Publication No. WO 2015/072305 discloses a multi-step solvent extraction method for purifying mixtures of VF2 and HFC-23 to isolate VF2 therefrom.

U.S. Patent No. 4,818,513 discloses methods for separating HF from a mixture with VF2 using a liquid organic barrier material (*e.g*., 1,1,1-trifluoroethane) to partially condense the feedstream.

U.S. Patent No. 3,381,041 discloses methods of purifying impure C1-C9 fluorinated alkanes and/or C2-C9 fluorinated alkenes using sulfuric acid and mercury/mercury-containing compounds.

There remains a need to find viable alternatives for applications, while retaining performance properties and environmental sustainability. The present disclosure identifies compositions comprising VF2 that can at least partially address these issues, as well as methods of making and purifying such compositions and methods of using such compositions, *e.g*., in etching applications.

### SUMMARY

Compositions comprising fluorochemicals such as VF2 (HFO-1132a) can have multiple applications/uses. As such, their manufacture and/or purification may benefit from stricter or more flexible requirements, depending upon the nature of the application/use. For instance, a composition that is manufactured/purified as a (co)monomer for polymerization probably has different tolerances for specific impurities than a composition that is manufactured/purified as an etchant gas. In a particular example, because the etchant gas purity requirements can tend to be more highly restrictive, a composition that is manufactured/purified as a (co)monomer for polymerization may be considered a relatively impure VF2 (HFO-1132a) composition for etchant gas purposes, and the additional purification needed to meet such restrictive purity requirements may be needed to render what could therefore be considered a relatively pure VF2 (HFO-1132a) composition. Such compositions, both relatively impure and relatively pure, can be characterized not only by components and their respective contents, but also (or alternatively) by functional characterizations that typically do not inherently obey structure-property correlations, at least not strictly.

The present disclosure deals with relatively impure and relatively pure VF2-based compositions, regardless of the exemplary components/characterizations. The present disclosure also identifies purification steps for relatively impure compositions, which can advantageously be linked to provision of increased purity and transformation into relatively pure compositions.

In addition, as noted, the present disclosure also provides for methods of using the compositions disclosed herein, in particular vis-a-vis formation of a plasma for enabling selective etching (and/or passivation) of substrates such as multilayer substrates with multilayer features.

### DETAILED DESCRIPTION

As used herein, a range of values referencing real numbers [A] and [B], expressed as "between [A] and [B]", should be understood as including [A] and [B] unless otherwise specified for either/both of [A] and [B], the same as if the range were expressed as "from [A] to [B]" or "at least [A]" or "up to [B]".

When reference is made to Group [Q] metals, transition metals, rare earth metals, and metalloids herein, it should be understood that these are reference to the Periodic Table of Elements, with Q being the (one or more) column(s) in the Periodic Table categorizing said metals.

Any description herein, even if described in relation to one or some specific embodiment(s), should be understood to be applicable to and interchangeable with other embodiments described or contemplated herein, to the extent that such application and/or interchangeability is not inoperable or specifically precluded.

### Compositions

Compositions according to the invention may be relatively impure or relatively pure, but in any case are based on a predominant content of 1,1-difluoroethylene (HFO-1132a; *aka* vinylidene fluoride, or VF2). Relatively impure compositions can comprise at least 70 wt% of VF2, *e.g.,* at least 80 wt%, at least 90 wt%, at least 95 wt%, at least 97 wt%, at least 99 wt%, at least 99.5 wt%, at least 99.7 wt%, at least 99.8 wt%, at least 99.9 wt%, at least 99.93 wt%, at least 99.95 wt%, or at least 99.97 wt%, based on a total weight of the composition. Additionally or alternatively, relatively impure compositions can comprise up to 99 wt% of VF2, *e.g.,* up to 99.5 wt%, up to 99.7 wt%, up to 99.8 wt%, up to 99.9 wt%, up to 99.93 wt%, up to 99.95 wt%, up to 99.97 wt%, or up to 99.99 wt%, based on a total weight of the composition. In particular, such compositions can comprise from 70 wt% to 99.97 wt% VF2, from 90 wt% to 99.97 wt% VF2, at least 99.5 wt% VF2, at least 99.9 wt% VF2, from 99.5 wt% to 99.99 wt% VF2, based on a total weight of the composition.

The remainder of the relatively impure composition may contain, but may not necessarily be limited to, one, some, or all of the following impurities: difluoromethane (HFC-32), 1,1-difluoroethane (HFC-152a), 1,2-difluoroethane (HFC-152), 1,2-difluoroethylene (HFO-1132, both cis- and trans- isomers), trifluoroethylene (HFO-1123), tetrafluoromethane (HFC-14), trifluoromethane (HFC-23), fluoromethane (HFC-41), fluoroethylene (HFO-1141), tetrafluoroethylene (HFO-1114), 3,3,3-trifluoro-1-propene (HFC-1243zf), 2,3,3,3-tetrafluoro-1-propene (HFO-1234yf), 1,3,3,3-tetrafluoro-1-propene (HFO-1234ze), hexafluoropropene (HFO-1216), fluoroethane (HFC-161), 1,1,1-trifluoroethane (HFC-143a), 1,1,2-trifluoroethane (HFC-143), 1,1,1,2-tetrafluoroethane (HFC-134a), 1,1,2,2-tetrafluoroethane (HFC-134), pentafluoroethane (HFC-125), hexafluoroethane (HFC-116), organic compounds containing a chlorine, bromine, and/or iodine atom, methane, ethane, ethylene, acetylene, propane, propylene, butane, isobutane, Group 1 metals, Group 2 metals, Group 13 metals, Group 14 metals, Group 15 metals, transition metals, rare earth metals, metalloids, carbon dioxide, ammonia, hydrogen fluoride, hydrogen chloride, chlorine, and water. The term "impurities," as used herein, should not be understood to necessarily imply a negative connotation - in some situations, components identified as impurities may have a positive or even synergistic effect in certain applications. Nevertheless, as the main/majority component of the compositions according to the present disclosure is VF2, all other compounds are identified herein as "impurities" for simplicity only and with no intent to limit the disclosure (or claims).

Particularly in, but not limited to, embodiments in which the composition comprises at least 99.5 wt% VF2, the VF2 compositions can contain: at most 3000 wppm (parts per million by weight) of a combination of difluoromethane (HFC-32), 1,1-difluoroethane (HFC-152a), 1,2-difluoroethane (HFC-152), 1,2-difluoroethylene (HFO-1132, both cis- and trans-isomers), and trifluoroethylene (HFO-1123); at most 1000 wppm of a combination of tetrafluoromethane (HFC-14), trifluoromethane (HFC-23), and fluoromethane (HFC-41); at most 1000 wppm of a combination of fluoroethylene (HFO-1141), tetrafluoroethylene (HFO-1114), 3,3,3-trifluoro-1-propene (HFC-1243zf), 2,3,3,3-tetrafluoro-1-propene (HFO-1234yf), 1,3,3,3-tetrafluoro-1-propene (HFO-1234ze), and hexafluoropropene (HFO-1216); at most 500 wppm of all organic compounds containing a chlorine, bromine, and/or iodine atom; at most 500 wppm of a combination of fluoroethane (HFC-161), 1,1,1-trifluoroethane (HFC-143a), 1,1,2-trifluoroethane (HFC-143), 1,1,1,2-tetrafluoroethane (HFC-134a), 1,1,2,2-tetrafluoroethane (HFC-134), pentafluoroethane (HFC-125), and hexafluoroethane (HFC-116); at most 500 wppm of a combination of methane, ethane, ethylene, acetylene, propane, and propylene; at most 500 wppm of a combination of Group 1 metals, Group 2 metals, Group 13 metals, Group 14 metals, Group 15 metals, transition metals, rare earth metals, and metalloids; and at most 1000 wppm of a combination of carbon dioxide, ammonia, hydrogen fluoride, hydrogen chloride, chlorine, and water.

As referenced herein, organic compounds containing a chlorine atom can encompass HCOs, HCCs, and HCFCs, among other organic compounds. Such chlorine-containing organic compounds specifically do not include, *e.g.,* HCl, HClO and salts thereof (such as sodium), HClO₄ and salts thereof (such as lithium, sodium, and/or potassium), SCl₂, SOCl₂, BCl₃, SiCl₄, H₂SiCl₂, and the like, and combinations thereof. Non-limiting examples of organic compounds containing a chlorine atom can include chloromethane (40), chlorofluoromethane (31), dichloromethane (30), chlorodifluoromethane (21), fluorodichloromethane (22), trichloromethane (20), chloro-trifluoromethane (13), dichlorodifluoromethane (12), fluorotrichloromethane (11), chloroethane (160), dichloroethanes

(150), chlorofluoroethanes (151), trichloroethanes (140), fluorodichloroethanes (141), chlorodifluoroethanes (142), tetrachloroethanes (130), fluorotrichloroethanes (131), diflourodichloroethanes (132), chlorotrifluoroethanes (133), 1-chloro-1-fluoroethylene (1131b), 1-chloro-2-fluoroethylene (1131d), 1,1-dichloroethylene (1130a), 1,2-dichloroethylene (1130), 2-chloro-1,1-difluoroethylene (1122c), 1-chloro-1,2-difluoroethylene (1122b), 1-chloro-3,3,3-trifluoropropene (1233zd), 2-chloro-3,3,3-trifluoropropene (1233xf), and the like, and combinations thereof. Although trifluoroiodomethane is one particular example of an organic compound containing an iodine atom, it should be understood by those of ordinary skill in the art that other examples can include, but are not necessarily limited to, iodo- analogs of the chloro-organics enumerated herein. The same bromo-analogs apply as non-limiting examples as well.

Relatively impure VF2 compositions may be characterized by a 100-year global warming potential (GWP) of at least 0.06, *e.g.,* at least 0.07, at least 0.08, at least 0.09, at least 0.10, at least 0.12, at least 0.14, at least 0.16, at least 0.18, at least 0.20, at least 0.25, at least 0.30, at least 0.35, at least 0.40, at least 0.45, or at least 0.50. Additionally or alternatively, relatively impure VF2 compositions may be characterized by a GWP of at most 30, *e.g.,* at most 25, at most 20, at most 15, at most 10, at most 8.0, at most 7.0, at most 6.0, at most 5.0, at most 4.0, at most 3.0, at most 2.5, at most 2.0, at most 1.5, at most 1.2, at most 1.0, at most 0.80, at most 0.60, at most 0.50, at most 0.40, or at most 0.30. In particular, the GWP of relatively impure VF2 compositions can be at least 0.07, from 0.06 to 30, from 0.06 to 1.0, from 0.07 to 20, or from 0.06 to 0.50.

Relatively impure VF2 compositions may be characterized by wideband spark ignition energies at various proportions of VF2 (plus impurities) in air, such as one, some, or all of the following: a wideband spark ignition energy at about 5.75 vol% of the composition in air of at least 130 mJ, *e.g.,* at least 140 mJ, optionally up to 180 mJ; a wideband spark ignition energy at about 5.50 vol% of the composition in air of at least 240 mJ, *e.g.*, at least 270 mJ, optionally up to 340 mJ; a wideband spark ignition energy at about 5.25 vol% of the composition in air of at least 450 mJ, *e.g.*, at least 520 mJ, optionally up to 600 mJ; and a wideband spark ignition energy at about 5.00 vol% of the composition in air of at least 670 mJ, *e.g.*, at least 800 mJ, optionally up to 900 mJ. Additionally or alternatively, these wideband spark ignition energies may be characteristics of relatively pure VF2 compositions. Wideband spark ignition energies, as described herein, can be measured using equipment and under conditions described in U.S. Provisional Patent Application No. 63/809,231, filed on May 20, 2025, the entire contents of which are incorporated herein, and the relevant contents of which are explicitly referenced to enable the aforementioned wideband spark ignition energies described herein. For example, said ignition energies refer to measurement at a temperature of ~23°C, a pressure of ~14.7 psia, and a volume of ~20L.

Relatively pure compositions can comprise at least 99.9 wt%, of VF2, *e.g.*, at least 99.93 wt%, at least 99.95 wt%, at least 99.97 wt%, at least 99.99 wt%, at least 99.995 wt%, at least 99.999 wt%, or at least 99.9995 wt%, based on a total weight of the composition. Additionally or alternatively, relatively pure compositions can comprise up to 100 wt% of VF2, *e.g.*, up to 99.99995 wt%, up to 99.9999 wt%, up to 99.9995 wt%, up to 99.999 wt%, up to 99.997 wt%, up to 99.995 wt%, up to 99.993 wt%, or up to 99.99 wt%, based on a total weight of the composition. In particular, such compositions can comprise from 99.9 wt% to 99.9999 wt% VF2, from 90 wt% to 99.97 wt% VF2, at least 99.5 wt% VF2, at least 99.9 wt% VF2, from 99.5 wt% to 99.99 wt% VF2, based on a total weight of the composition.

Particularly in, but not limited to, embodiments in which the composition comprises at least 99.9 wt% VF2, whether as relatively impure or as relatively pure compositions, the VF2 compositions can comprise: at most 800 wppm of a combination of difluoromethane (HFC-32), 1,1-difluoroethane (HFC-152a), 1,2-difluoroethane (HFC-152), 1,2-difluoroethylene (HFO-1132, both cis- and trans- isomers), and trifluoroethylene (HFO-1123); at most 100 wppm of a combination of tetrafluoromethane (HFC-14), trifluoromethane (HFC-23), fluoromethane (HFC-41); at most 200 wppm of a combination of fluoroethylene (HFO-1141), tetrafluoroethylene (HFO-1114), 3,3,3-trifluoro-1-propene (HFC-1243zf), 2,3,3,3-tetrafluoro-1-propene (HFO-1234yf), 1,3,3,3-tetrafluoro-1-propene (HFO-1234ze), and hexafluoropropene (HFO-1216); at most 50 wppm of all organic compounds containing a chlorine, bromine, and/or iodine atom; at most 50 wppm of a combination of fluoroethane (HFC-161), 1,1,1-trifluoroethane (HFC-143a), 1,1,2-trifluoroethane (HFC-143), 1,1,1,2-tetrafluoroethane (HFC-134a), 1,1,2,2-tetrafluoroethane (HFC-134), pentafluoroethane (HFC-125), and hexafluoroethane (HFC-116); at most 70 wppm of a combination of methane, ethane, ethylene, acetylene, propane, and propylene; at most 50 wppm of a combination of Group 1 metals, Group 2 metals, Group 13 metals, Group 14 metals, Group 15 metals, transition metals, rare earth metals, and metalloids; and at most 100 wppm of a combination of carbon dioxide, ammonia, hydrogen fluoride, hydrogen chloride, chlorine, and water.

Particularly in, but not limited to, embodiments in which the composition comprises at least 99.99 wt% VF2, whether as relatively impure or as relatively pure compositions, the VF2 compositions can comprise: at most 100 wppm of a combination of difluoromethane (HFC-32), 1,1-difluoroethane (HFC-152a), 1,2-difluoroethane (HFC-152), 1,2-difluoroethylene (HFO-1132, both cis- and trans- isomers), trifluoroethylene (HFO-1123), tetrafluoromethane (HFC-14), trifluoromethane (HFC-23), fluoromethane (HFC-41), fluoroethylene (HFO-1141), tetrafluoroethylene (HFO-1114), 3,3,3-trifluoro-1-propene (HFC-1243zf), 2,3,3,3-tetrafluoro-1-propene (HFO-1234yf), 1,3,3,3-tetrafluoro-1-propene (HFO-1234ze), hexafluoropropene (HFO-1216), fluoroethane (HFC-161), 1,1,1-trifluoroethane (HFC-143a), 1,1,2-trifluoroethane (HFC-143), 1,1,1,2-tetrafluoroethane (HFC-134a), 1,1,2,2-tetrafluoroethane (HFC-134), pentafluoroethane (HFC-125), hexafluoroethane (HFC-116), all organic compounds containing a chlorine, bromine, and/or iodine atom, methane, ethane, ethylene, acetylene, propane, propylene, Group 1 metals, Group 2 metals, Group 13 metals, Group 14 metals, Group 15 metals, transition metals, rare earth metals, metalloids, carbon dioxide, ammonia, hydrogen fluoride, hydrogen chloride, chlorine, and water.

Relatively pure VF2 compositions may be characterized by a 100-year global warming potential (GWP) of at most 0.80, *e.g.*, at most 0.75. at most 0.70, at most 0.65, at most 0.60, at most 0.55, at most 0.50, at most 0.45, at most 0.40, at most 0.35, at most 0.30, at most 0.25, at most 0.20, at most 0.18, at most 0.16, at most 0.14, at most 0.12, at most 0.11, at most 0.10, at most 0.095, at most 0.090, at most 0.085, at most 0.080, at most 0.075, at most 0.070, at most 0.065, at most 0.060, at most 0.055, at most 0.052, or at most 0.050. Additionally or alternatively, relatively pure VF2 compositions may be characterized by a GWP of at least 0.010, *e.g.*, at least 0.015, at least 0.020, at least 0.025, at least 0.030, at least 0.035, at least 0.040, at least 0.045, at least 0.050, at least 0.052, at least 0.055, at least 0.060, or at least 0.065. In particular, the GWP of relatively pure VF2 compositions can be at most 0.055, at most 0.060, from 0.010 to 0.055, from 0.025 to 0.060, or from 0.030 to 0.052.

### Methods of Making/Purifying Compositions

Although there are more detailed methods for making a composition comprising 1,1-difluoroethylene (HFO-1132a; VF2) as the sole desired product, the present disclosure focuses on starting with VF2 compositions that are less pure than desired, referred to herein as "relatively impure," and purifying them to reduce impurities (all other less desirable or undesirable components). As such, a method for purifying a relatively impure VF2 composition can comprise a step of providing a relatively impure VF2 composition according to the present disclosure. Such a composition can comprise: a first content of VF2 (*e.g.*, from 70 wt% to 99.99 wt%, based on a total weight of the composition). Other components of such composition can include one or more of: a first content of difluoromethane (HFC-32); a first content of 1,2-difluoroethylene (HFO-1132, collectively cis- and trans- isomers); a first content of difluoroethane (HFC-152, collectively both 1,1- and 1,2- isomers); a first content of trifluoromethane (HFC-23); a first content of fluoromethane (HFC-41); a first content, collectively, of all organic compounds containing a chlorine, bromine, and/or iodine atom; a first content of fluoroethylene (HFO-1141); a first content of hexafluoropropene (HFO-1216); a first content of fluoroethane (HFC-161); a first content of trifluoroethane (HFC-143, collectively both 1,1,1- and 1,1,2- isomers); a first content of tetrafluoroethane (HFC-134, collectively 1,1,1,2- and 1,1,2,2- isomers); a first content of pentafluoroethane (HFC-125); a first content of hexafluoroethane (HFC-116); a first combined content of ethylene and propylene; and a first combined content of water. Additionally or alternatively, there may be first contents of other impurities that are enumerated in the above list.

In a subsequent step, the relatively impure VF2 composition can be exposed to a purification process under conditions so as to form a relatively pure VF2 composition. The relatively pure VF2 composition can comprise: a second content of VF2 (*e.g.*, from 99.9 wt% to 99.9999 wt%, based on a total weight of the relatively pure composition) that is at least 0.1 wt% greater than the corresponding first content of VF2 in the relatively impure composition. Other components of such composition can include one or more of: a second content of difluoromethane (HFC-32); a second content of 1,2-difluoroethylene (HFO-1132, collectively cis- and trans- isomers); a second content of difluoroethane (HFC-152, collectively both 1,1- and 1,2- isomers); a second content of trifluoromethane (HFC-23); a second content of fluoromethane (HFC-41); a second content, collectively, of all organic compounds containing a chlorine, bromine, and/or iodine atom; a second content of fluoroethylene (HFO-1141); a second content of hexafluoropropene (HFO-1216); a second content of fluoroethane (HFC-161); a second content of trifluoroethane (HFC-143, collectively both 1,1,1- and 1,1,2- isomers); a second content of tetrafluoroethane (HFC-134, collectively 1,1,1,2- and 1,1,2,2- isomers); a second content of pentafluoroethane (HFC-125); a second content of hexafluoroethane (HFC-116); a second combined content of ethylene and propylene; and a second combined content of water. Additionally or alternatively, there may be second contents of other impurities that are enumerated in the above list but that correspond to identical impurities in the relatively impure VF2 composition. In an advantageous embodiment, the second content of difluoromethane (HFC-32) is at least 25% lower, *e.g.*, at least 30% lower, at least 40% lower, or at least 50% lower, than its corresponding first content. Optionally at least one other of said second contents and second combined contents can additionally be at least 25% lower, *e.g.*, at least 30% lower, at least 40% lower, or at least 50% lower, than its corresponding first content. Additionally or alternatively, the relatively pure VF2 composition from the purification process can comprise no second content or second combined content that is more than 30 wppm higher, or more than 200% higher, than its corresponding first content or first combined content.

In advantageous embodiments, the purification process can comprise a distillation step to capture overhead/gas-phase product, a distillation step to capture bottoms/liquid and/or solid phase product, an azeotropic distillation step, an absorption step (inclusive of absorption and adsorption, individually or in combination), a drying and/or solvent removal step, an extraction step, a filtration step, a membrane separation step, or a combination thereof. Any one or more of these steps may be performed under supercritical conditions. In particular, the purification process can comprise an absorption step, a distillation step (not azeotropic), or a combination thereof.

Without meaning to be limiting, possible/exemplary absorptive media can include, but are not necessarily limited to, aluminates, aluminum oxide hydroxides, aluminosilicates, hydroxylated aluminosilicates, aluminosilicate gels, silicates, silicon oxide hydroxide, silica gels, diatomaceous earth, zeolites (typically with 8-ring or smaller apertures), MOFs (metal organic frameworks), ZIFs (zeolite imidazolate frameworks), molecular sieves, activated carbon, graphene, amorphous carbon, naturally-occurring and/or modified clays, intercalated clays, ionic liquids, polymer particles, doped and/or supported versions thereof, and the like, and combinations thereof.

Although it is possible to immediately put the relatively pure VF2 composition to work immediately in a desired use/application, such relatively pure VF2 compositions can optionally but typically be isolated and/or stored at least temporarily, and perhaps even shipped (in appropriate vessels), for future use in its desired application(s).

### Applications/Uses of Compositions

A desired use of the relatively pure VF2 compositions according to the present disclosure is as an etchant gas, such as in a plasma etching process. As such, methods according to the invention may further include passing the relatively pure VF2 composition, directly and/or from isolation/storage, to a plasma etching process.

A multilayered substrate can be provided, which comprises one or more features such as trenches, slits, vias, through-holes, staircase contacts, capacitor holes, contact holes, connectors, or the like, or other features spanning more than one layer. Such substrates, and specifically such features, can be selectively etched. Such substrates to be etched are typically placed or located in an etching chamber.

Without meaning to be limiting, typical substrates can include or be semiconductor wafers, or portions thereof. For example, the substrate can advantageously comprise silicon, and at least one of the layers can comprise silicon nitride, silicon oxide, silicon oxynitride, crystalline Si, polycrystalline silicon, amorphous silicon, polysilicon, silicon oxide hydroxide, silicon carbide, carbon-containing silicon oxide, graphene, amorphous carbon, or other carbonaceous conductive and/or dielectric materials, optionally other metals and/or metalloids in their oxide, nitride, oxynitride, carbide, or other forms, and combinations thereof.

The relatively pure VF2 composition can be supplied to the etching chamber. Immediately prior to or during this supply step, the relatively pure VF2 composition can be converted into a plasma. Although it is possible to create a plasma with the etchant gas alone, typically generation of the plasma involves adding: an inert gas, such as comprising helium, nitrogen, neon, argon, krypton, xenon, or a combination thereof; a reactive gas such as comprising hydrogen and/or a source of hydrogen, carbon monoxide, carbon dioxide, carbonyl sulfide, nitric oxide, nitrogen oxide, nitrous oxide, oxygen, ozone, hydrogen sulfide, sulfur dioxide, or a combination thereof; or both such gases.

In one embodiment, the etching conditions can be cryogenic, which can include temperatures from 0°C to -196°C in or on a wall of the etching chamber, *e.g*., from 0°C to -175°C, from 0°C to -150°C, from 0°C to -125°C, from 0°C to -110°C, from 0°C to -100°C, from 0°C to -90°C, from 0°C to -80°C, from 0°C to -70°C, from 0°C to -60°C, from 0°C to - 50°C, from 0°C to -40°C, from -5°C to -196°C, from -5°C to -175°C, from -5°C to -150°C, from -5°C to -125°C, from -5°C to -110°C, from -5°C to -100°C, from -5°C to -90°C, from - 5°C to -80°C, from -5°C to -70°C, from -5°C to -60°C, from -5°C to -50°C, from -5°C to - 40°C, from -10°C to -196°C, from -10°C to -175°C, from -10°C to -150°C, from -10°C to - 125°C, from -10°C to -110°C, from -10°C to -100°C, from -10°C to -90°C, from -10°C to - 80°C, from -10°C to -70°C, from -10°C to -60°C, from -10°C to -50°C, from -10°C to -40°C, from -20°C to -196°C, from -20°C to -175°C, from -20°C to -150°C, from -20°C to -125°C, from -20°C to -110°C, from -20°C to -100°C, from -20°C to -90°C, from -20°C to -80°C, from -10°C to -70°C, from -20°C to -60°C, from -20°C to -50°C, from -20°C to -40°C, from -30°C to -196°C, from -30°C to -175°C, from -30°C to -150°C, from -30°C to -125°C, from -30°C to -110°C, from -30°C to -100°C, from -30°C to -90°C, from -30°C to -80°C, from -30°C to - 70°C, from -30°C to -60°C, from -30°C to -50°C, from -40°C to -196°C, from -40°C to -175°C, from -40°C to -150°C, from -40°C to -125°C, from -40°C to -110°C, from -10°C to -100°C, from -10°C to -90°C, from -40°C to -80°C, from -40°C to -70°C, from -40°C to -60°C, from - 50°C to -196°C, from -50°C to -175°C, from -50°C to -150°C, from -50°C to -125°C, from - 50°C to -110°C, from -50°C to -100°C, from -50°C to -90°C, from -50°C to -80°C, from -75°C to -196°C, from -75°C to -175°C, from -75°C to -150°C, from -75°C to -125°C, from -75°C to -110°C, from -75°C to -100°C, from -100°C to -196°C, from -100°C to -175°C, from -100°C to -150°C, from -100°C to -125°C, or from -125°C to -196°C. In particular, the cryogenic temperature can be from 0°C to -196°C, from -5°C to -80°C, from -10°C to -100°C, or from - 30°C to -196°C.

In certain embodiments, the plasma can be generated and/or maintained using an energy from 5 eV to 90 eV, *e.g.,* from 5 eV to 75 eV, from 5 eV to 70 eV, from 5 eV to 60 eV, from 5 eV to 50 eV, from 5 eV to 45 eV, from 5 eV to 40 eV, from 5 eV to 30 eV, from 5 eV to 25 eV, from 5 eV to 20 eV, from 10 eV to 90 eV, from 10 eV to 75 eV, from 10 eV to 70 eV, from 10 eV to 60 eV, from 10 eV to 50 eV, from 10 eV to 45 eV, from 10 eV to 40 eV, from 10 eV to 30 eV, from 10 eV to 25 eV, from 10 eV to 20 eV, from 15 eV to 90 eV, from 15 eV to 75 eV, from 15 eV to 70 eV, from 15 eV to 60 eV, from 15 eV to 50 eV, from 15 eV to 45 eV, from 15 eV to 40 eV, from 15 eV to 30 eV, from 15 eV to 25 eV, from 20 eV to 90 eV, from 20 eV to 75 eV, from 20 eV to 70 eV, from 20 eV to 60 eV, from 20 eV to 50 eV, from 20 eV to 45 eV, from 20 eV to 40 eV, from 20 eV to 30 eV, from 25 eV to 90 eV, from 25 eV to 75 eV, from 25 eV to 70 eV, from 25 eV to 60 eV, from 25 eV to 50 eV, from 25 eV to 45 eV, from 25 eV to 40 eV, from 40 eV to 90 eV, from 40 eV to 75 eV, from 40 eV to 70 eV, from 40 eV to 60 eV, from 50 eV to 90 eV, from 50 eV to 75 eV, from 50 eV to 70 eV, from 60 eV to 90 eV, from 60 eV to 75 eV, or from 70 eV to 90eV. In particular, the energy can be from 5 eV to 75 eV, from 10 eV to 40 eV, from 15 eV to 70 eV, or from 5 eV to 50 eV.

The plasma formed from the relatively pure VF2 compositions can be allowed (enabled) to selectively etch one or more of the features such as trenches, slits, vias, through-holes, staircase contacts, capacitor holes, contact holes, connectors, or other features spanning more than one layer of the multilayered substrate. Although such features can be of any size and/or dimensional ratio (*e.g*., diameter-to-depth, shortest planar dimension-to-depth, etc.), particular attention is paid to those features having dimensional ratios from 3:1 to 2500:1, *e.g.*, 5:1 to 1000:1, 5:1 to 500:1, or 5:1 to 100:1.

Optionally, but typically, the plasma formed from the relatively pure VF2 compositions can also (simultaneously) passivate one or more surfaces of the multilayer substrate and/or of one or more of its features (including those that are etched).

Without being bound by theory, the relatively pure VF2 compositions according to the invention, when converted into a plasma such as using an aforementioned energy range and/or whose chamber is controlled to exhibit an aforementioned cryogenic temperature in and/or on its wall(s), optionally as supplemented by one or more inert and/or reactive gases, may form one or more fragments that enable particularly desirable selective etch and/or passivation characteristics for particular substrates and/or generally. Non-limiting examples of such fragments may include F, F+, C+, H₂, HC, HC+, CF, CF+, F₂, F₂+, H₂C, H₂C+, HC₂, HC₂+, HCF, HCF+, C₂F, C₂F+, CF₂, CF₂+, HF₂+, H₂C₂, H₂C₂+, H₂CF, H₂CF+, HC₂F, HC₂F+, HCF₂, HCF₂+, C₂F₂, C₂F₂+, H₂C₂F, H₂C₂F+, H₂CF₂, H₂CF₂+, HC₂F₂, HC₂F₂+, H₂C₂F₂+, and combinations and degradation products thereof.

### Additional Embodiments

Additionally or alternatively, the present disclosure can include one or more of the following embodiments.

Embodiment 1. A composition comprising at least 99.5 wt% 1,1-difluoroethylene (HFO-1132a), based on a total weight of the composition, and one or more of the following impurities, based on a total weight of the composition: at most 3000 wppm (parts per million by weight) of a combination of difluoromethane (HFC-32), 1,1-difluoroethane (HFC-152a), 1,2-difluoroethane (HFC-152), 1,2-difluoroethylene (HFO-1132, both cis- and trans- isomers), and trifluoroethylene (HFO-1123); at most 1000 wppm of a combination of tetrafluoromethane (HFC-14), trifluoromethane (HFC-23), and fluoromethane (HFC-41); at most 1000 wppm of a combination of fluoroethylene (HFO-1141), tetrafluoroethylene (HFO-1114), 3,3,3-trifluoro-1-propene (HFC-1243zf), 2,3,3,3-tetrafluoro-1-propene (HFO-1234yf), 1,3,3,3-tetrafluoro-1-propene (HFO-1234ze), and hexafluoropropene (HFO-1216); at most 500 wppm of all organic compounds containing a chlorine, bromine, and/or iodine atom; at most 500 wppm of a combination of fluoroethane (HFC-161), 1,1,1-trifluoroethane (HFC-143a), 1,1,2-trifluoroethane (HFC-143), 1,1,1,2-tetrafluoroethane (HFC-134a), 1,1,2,2-tetrafluoroethane (HFC-134), pentafluoroethane (HFC-125), and hexafluoroethane (HFC-116); at most 500 wppm of a combination of methane, ethane, ethylene, acetylene, propane, propylene, butane, and isobutane; at most 500 wppm of a combination of Group 1 metals, Group 2 metals, Group 13 metals, Group 14 metals, Group 15 metals, transition metals, rare earth metals, and metalloids; and at most 1000 wppm of a combination of carbon dioxide, ammonia, hydrogen fluoride, hydrogen chloride, chlorine, and water.

Embodiment 2. The composition of embodiment 1, further comprising one or more of: a GWP of at least 0.06, optionally at most 30; a wideband spark ignition energy at about 5.75 vol% of the composition in air of at least 130 mJ; a wideband spark ignition energy at about 5.50 vol% of the composition in air of at least 240 mJ; a wideband spark ignition energy at about 5.25 vol% of the composition in air of at least 450 mJ; and a wideband spark ignition energy at about 5.00 vol% of the composition in air of at least 670 mJ.

Embodiment 3. The composition of embodiment 1 or embodiment 2, further comprising one or more of: a GWP from 0.06 to 1.0; a wideband spark ignition energy at about 5.75 vol% of the composition in air of at most 140 mJ; a wideband spark ignition energy at about 5.50 vol% of the composition in air of at most 270 mJ; a wideband spark ignition energy at about 5.25 vol% of the composition in air of at least 520 mJ; and a wideband spark ignition energy at about 5.00 vol% of the composition in air of at least 800 mJ.

Embodiment 4. The composition of any of embodiments 1-3, wherein the composition comprises at least 99.9 wt% 1,1-difluoroethylene (HFO-1132a), based on a total weight of the composition, and one or more of the following impurities, based on a total weight of the composition: at most 800 wppm of a combination of difluoromethane (HFC-32), 1,1-difluoroethane (HFC-152a), 1,2-difluoroethane (HFC-152), 1,2-difluoroethylene (HFO-1132, both cis- and trans- isomers), and trifluoroethylene (HFO-1123); at most 100 wppm of a combination of tetrafluoromethane (HFC-14), trifluoromethane (HFC-23), fluoromethane (HFC-41); at most 200 wppm of a combination of fluoroethylene (HFO-1141), tetrafluoroethylene (HFO-1114), 3,3,3-trifluoro-1-propene (HFC-1243zf), 2,3,3,3-tetrafluoro-1-propene (HFO-1234yf), 1,3,3,3-tetrafluoro-1-propene (HFO-1234ze), and hexafluoropropene (HFO-1216); at most 50 wppm of all organic compounds containing a chlorine, bromine, and/or iodine atom; at most 50 wppm of a combination of fluoroethane (HFC-161), 1,1,1-trifluoroethane (HFC-143a), 1,1,2-trifluoroethane (HFC-143), 1,1,1,2-tetrafluoroethane (HFC-134a), 1,1,2,2-tetrafluoroethane (HFC-134), pentafluoroethane (HFC-125), and hexafluoroethane (HFC-116); at most 70 wppm of a combination of methane, ethane, ethylene, acetylene, propane, propylene, butane, and isobutane; at most 50 wppm of a combination of Group 1 metals, Group 2 metals, Group 13 metals, Group 14 metals, Group 15 metals, transition metals, rare earth metals, and metalloids; and at most 100 wppm of a combination of carbon dioxide, ammonia, hydrogen fluoride, hydrogen chloride, chlorine, and water.

Embodiment 5. The composition of any of embodiments 1-4, wherein the composition comprises at least 99.99 wt% 1,1-difluoroethylene (HFO-1132a), based on a total weight of the composition, and at most 100 wppm of a combination of difluoromethane (HFC-32), 1,1-difluoroethane (HFC-152a), 1,2-difluoroethane (HFC-152), 1,2-difluoroethylene (HFO-1132, both cis- and trans- isomers), trifluoroethylene (HFO-1123), tetrafluoromethane (HFC-14), trifluoromethane (HFC-23), fluoromethane (HFC-41), fluoroethylene (HFO-1141), tetrafluoroethylene (HFO-1114), 3,3,3-trifluoro-1-propene (HFC-1243zf), 2,3,3,3-tetrafluoro-1-propene (HFO-1234yf), 1,3,3,3-tetrafluoro-1-propene (HFO-1234ze), hexafluoropropene (HFO-1216), fluoroethane (HFC-161), 1,1,1-trifluoroethane (HFC-143a), 1,1,2-trifluoroethane (HFC-143), 1,1,1,2-tetrafluoroethane (HFC-134a), 1,1,2,2-tetrafluoroethane (HFC-134), pentafluoroethane (HFC-125), hexafluoroethane (HFC-116), all organic compounds containing a chlorine, bromine, and/or iodine atom, methane, ethane, ethylene, acetylene, propane, propylene, butane, isobutane, Group 1 metals, Group 2 metals, Group 13 metals, Group 14 metals, Group 15 metals, transition metals, rare earth metals, metalloids, carbon dioxide, ammonia, hydrogen fluoride, hydrogen chloride, chlorine, and water.

Embodiment 6. A method for purifying a relatively impure 1,1-difluoroethylene (HFO-1132a) composition comprising: providing a relatively impure 1,1-difluoroethylene (HFO-1132a) composition comprising: a first content from 70 wt% to 99.99 wt% of 1,1-difluoroethylene (HFO-1132a), based on a total weight of the composition; a first content of difluoromethane (HFC-32); a first content of 1,2-difluoroethylene (HFO-1132, collectively cis- and trans- isomers); a first content of difluoroethane (HFC-152, collectively both 1,1- and 1,2-isomers); a first content of trifluoromethane (HFC-23); a first content of fluoromethane (HFC-41); a first content, collectively, of all organic compounds containing a chlorine, bromine, and/or iodine atom; a first content of fluoroethylene (HFO-1141); a first content of hexafluoropropene (HFO-1216); a first content of fluoroethane (HFC-161); a first content of trifluoroethane (HFC-143, collectively both 1,1,1- and 1,1,2- isomers); a first content of tetrafluoroethane (HFC-134, collectively 1,1,1,2- and 1,1,2,2- isomers); a first content of pentafluoroethane (HFC-125); a first content of hexafluoroethane (HFC-116); a first combined content of ethylene and propylene; and a first combined content of water; exposing the relatively impure 1,1-difluoroethylene (HFO-1132a) composition to a purification process under conditions so as to form a relatively pure 1,1-difluoroethylene (HFO-1132a) composition, wherein the relatively pure 1,1-difluoroethylene (HFO-1132a) composition comprises: a second content from 99.9 wt% to 99.9999 wt% of 1,1-difluoroethylene (HFO-1132a), based on a total weight of the relatively pure composition, which second content is at least 0.1 wt% greater than its corresponding first content; a second content of difluoromethane (HFC-32); a second content of 1,2-difluoroethylene (HFO-1132, collectively cis- and trans-isomers); a second content of difluoroethane (HFC-152, collectively both 1,1- and 1,2-isomers); a second content of trifluoromethane (HFC-23); a second content of fluoromethane (HFC-41); a second content, collectively, of all organic compounds containing a chlorine, bromine, and/or iodine atom; a second content of fluoroethylene (HFO-1141); a second content of hexafluoropropene (HFO-1216); a second content of fluoroethane (HFC-161); a second content of trifluoroethane (HFC-143, collectively both 1,1,1- and 1,1,2- isomers); a second content of tetrafluoroethane (HFC-134, collectively 1,1,1,2- and 1,1,2,2- isomers); a second content of pentafluoroethane (HFC-125); a second content of hexafluoroethane (HFC-116); a second combined content of ethylene and propylene; and a second combined content of water, wherein the second content of difluoromethane (HFC-32) and optionally at least one other of said second contents and second combined contents is(are) at least 25% lower than its(their) corresponding first content(s); and optionally isolating and/or storing said relatively pure 1,1-difluoroethylene (HFO-1132a) composition.

Embodiment 7. The method of embodiment 6, wherein the relatively pure 1,1-difluoroethylene (HFO-1132a) composition from the purification process comprises no second content or second combined content is more than 30 wppm or more than 200% higher than its corresponding first content or first combined content.

Embodiment 8. The method of embodiment 6 or embodiment 7, wherein the purification process comprises a distillation step to capture overhead/gas-phase product, a distillation step to capture bottoms/liquid and/or solid phase product, an azeotropic distillation step, an absorption step, a drying and/or solvent removal step, an extraction step, a filtration step, a membrane separation step, or a combination thereof.

Embodiment 9. The method of any one of embodiments 6-8, further comprising passing said relatively pure 1,1-difluoroethylene (HFO-1132a) composition to a plasma etching process.

Embodiment 10. The method of embodiment 9, wherein the plasma etching process comprises: providing a multilayered substrate comprising one or more trenches, slits, vias, through-holes, staircase contacts, capacitor holes, contact holes, connectors, or other features spanning more than one layer to be selectively etched in an etching chamber; supplying the relatively pure 1,1-difluoroethylene (HFO-1132a) composition to the etching chamber, immediately prior to or during which supplying step the relatively pure 1,1-difluoroethylene (HFO-1132a) composition is converted into a plasma; allowing the plasma to selectively etch one or more of the trenches, slits, vias, through-holes, staircase contacts, capacitor holes, contact holes, connectors, or other features spanning more than one layer of the multilayered substrate.

Embodiment 11. The method of embodiment 10, wherein the plasma also passivates one or more surfaces of the multilayer substrate and/or of one or more of its features.

Embodiment 12. The method of embodiment 10 or embodiment 11, wherein the substrate comprises silicon and wherein at least one of the layers comprises silicon nitride, silicon oxide, silicon oxynitride, crystalline Si, polycrystalline silicon, amorphous silicon, polysilicon, silicon oxide hydroxide, silicon carbide, carbon-containing silicon oxide, graphene, amorphous carbon, or other carbonaceous conductive and/or dielectric materials, optionally other metals and/or metalloids in their oxide, nitride, oxynitride, carbide, or other forms, and combinations thereof.

Embodiment 13. The method of any of embodiments 9-12, wherein generation of the plasma involves adding: an inert gas comprising helium, nitrogen, neon, argon, krypton, xenon, or a combination thereof; a reactive gas comprising hydrogen and/or a source of hydrogen, carbon monoxide, carbon dioxide, carbonyl sulfide, nitric oxide, nitrogen oxide, nitrous oxide, oxygen, ozone, hydrogen sulfide, sulfur dioxide, or a combination thereof; or both.

Embodiment 14. The method of any of embodiments 6-13, wherein the etching conditions include imposition of cryogenic temperatures ranging from -5°C to -80°C in or on a wall of the etching chamber.

Embodiment 15. The method of any of embodiments 6-14, wherein the plasma is generated and/or maintained using an energy from 5 eV to 75 eV.

The following Examples are intended to support and/or exemplify, but not necessarily limit, the scope of the invention, as recited in the claims. In this disclosure, the roots of the inclusive words "comprising", "containing", and "including" can alternatively encompass exclusionary or semi-exclusionary terms, such as variations of "consisting of" and/or "consisting essentially of", respectively, the latter of which should be understood to be given the meaning provided by United States case law as of the filing and/or priority date(s) hereof, as applicable.

The following Examples are intended to support and/or exemplify, but not necessarily limit, the scope of the invention, as recited in the claims. In this disclosure, the roots of the inclusive words "comprising" and "including" can alternatively include exclusionary or semi-exclusionary terms, such as variations of "consisting of" and/or "consisting essentially of", respectively, the latter of which should be understood to be given the meaning provided by United States case law.

### EXAMPLES

### Examples 1-6. Absorption of HFC-32 in HFO-1132a Composition

In each of these examples an HFO-1132a composition doped with varying amounts of HFC-32 was flowed through a column with an absorbent to test efficacy as a purification step.

For Examples 2-3 and 5-6, Siliporite^{™} G5 B1.6/2.5 (5A) molecular sieves (~1:1 silica:alumina molar ratio) commercially available from Arkema were loaded into a column and activated at ~120°C for ~16 hours at ~250 milliTorr pressure before use. For Example 1, an identical molecular sieve absorbent material was used, but activation was done at a lower temperature (~60) and for a shorter time (~4 hours). For Example 4, a zeolite (ZSM-5) absorbent (believed to have an ~38:1 silica:alumina ratio and a pore size of ~5 Angstroms) was purchased from ACS Material and used as received. Examples 1, 5, and 6 used a long path column (~1" long, ~1" OD), while each of Examples 2-4 used short path columns (each ~0.4' long, ~1" OD). In Examples 1, 5, and 6, ~66 g of absorbent was used, and ~20 grams of absorbent were used in each of Examples 2-4. Pressure in each column was ~30 psia, and ~100 mL/min of the HFO-1132a compositions were flowed through each column over a period of ~12-20 minutes.

All experiments were run on Agilent^{™} 8890B GC-MS system or a 7890B GC-MS/FID system using OpenLab CDS or OpenLab Chem Station software. The samples were injected manually using a gastight sampling syringe or using a gas sampling valve.

Examples 1-5 were run at ~30 psia and Example 6 was run at ~90 psia initially and then set at 30 psia for the collection of the last three samples, all at a ~100mL/min flow rate. Examples 1 and 5 each involved identical as-synthesized HFO-1132a compositions that were measured to contain ~0.117 mol% HFC-32. Examples 2 and 4 involved identical HFO-1132a compositions that were spiked with HFC-32 and intended to attain ~3.75 mol% HFC-32. Example 6 was overspiked with HFC-32, yielding ~22.4 mol% impurity. Example 3 involved an HFO-1132a composition that was spiked with HFC-32 to attain ~1.86 mol% HFC-32. These concentrations reflect zero (0) time points. In Example 1, collection occurred at three ~4-minute intervals up through ~12 minutes. In Examples 2-5, collection occurred at five ~3-minute intervals up through ~15 minutes. In Example 6, collection occurred at ~3-4-minute intervals up through ~20 minutes. Area integrations yielded the impurity results shown in Table 1 below.

**Table 1.**

| **Interval** | **Example 1 mol% 32** | **Example 2 mol% 32** | **Example 3 mol% 32** | **Example 4 mol% 32** | **Example 5 mol% 32** | **Example 6 mol% 32** |
|---|---|---|---|---|---|---|
| 0 | 0.117 | 3.75 | 1.86 | 1.86 | 0.117 | 22.4 |
| 1 | 0.092 | 0.733 | 0.164 | 2.91 | -- | 0.186 |
| 2 | 0.069 | 0.740 | 0.160 | -- | 0.003 | 0.164 |
| 3 | 0.064 | 1.08* | -- | 3.38 | 0.002 | 0.140 |
| 4 | -- | 1.22* | 0.186 | -- | 0.003 | 0.205 |
| 5 | -- | 1.58 | 0.211 | 3.45 | 0.000** | 0.349 |
| 6 | -- | -- | -- | -- | -- | 0.843 |

| | | | | | | |
|---|---|---|---|---|---|---|
| * represents arithmetic average of two analyses; ** represents less than 5 molar ppm HFC-32 measured. | | | | | | |

As can be seen from the data in Table 1, the 5A molecular sieves in Examples 1-3 and 5-6 seem to offer effective absorption of HFC-32, relative to HFO-1132a, validating a purification regime that includes an absorptive purification. Even very high levels of HFC-32 impurity in Example 6 seemed to be effectively mitigated by the absorbent material, which are of approximately the same order as the starting material of Example 5, indicating that a two-step (or multi-step) absorptive purification process would likely be quite effective. The zeolite of Example 4 seems to selectively absorb more HFO-1132a than HFC-32, making it a less desirable choice, as it appears to concentrate the impurity instead of the desired product.

## Claims

1. A composition comprising at least 99.5 wt% 1,1-difluoroethylene (HFO-1132a), based on a total weight of the composition, and one or more of the following impurities, based on a total weight of the composition:
at most 3000 wppm (parts per million by weight) of a combination of difluoromethane (HFC-32), 1,1-difluoroethane (HFC-152a), 1,2-difluoroethane (HFC-152), 1,2-difluoroethylene (HFO-1132, both cis- and trans- isomers), and trifluoroethylene (HFO-1123);
at most 1000 wppm of a combination of tetrafluoromethane (HFC-14), trifluoromethane (HFC-23), and fluoromethane (HFC-41);
at most 1000 wppm of a combination of fluoroethylene (HFO-1141), tetrafluoroethylene (HFO-1114), 3,3,3-trifluoro-1-propene (HFC-1243zf), 2,3,3,3-tetrafluoro-1-propene (HFO-1234yf), 1,3,3,3-tetrafluoro-1-propene (HFO-1234ze), and hexafluoropropene (HFO-1216);
at most 500 wppm of all organic compounds containing a chlorine, bromine, and/or iodine atom;
at most 500 wppm of a combination of fluoroethane (HFC-161), 1,1,1-trifluoroethane (HFC-143a), 1,1,2-trifluoroethane (HFC-143), 1,1,1,2-tetrafluoroethane (HFC-134a), 1,1,2,2-tetrafluoroethane (HFC-134), pentafluoroethane (HFC-125), and hexafluoroethane (HFC-116);
at most 500 wppm of a combination of methane, ethane, ethylene, acetylene, propane, propylene, butane, and isobutane;
at most 500 wppm of a combination of Group 1 metals, Group 2 metals, Group 13 metals, Group 14 metals, Group 15 metals, transition metals, rare earth metals, and metalloids; and
at most 1000 wppm of a combination of carbon dioxide, ammonia, hydrogen fluoride, hydrogen chloride, chlorine, and water.

2. The composition of claim 1, further comprising one or more of:
a GWP of at least 0.06, optionally at most 30;
a wideband spark ignition energy at about 5.75 vol% of the composition in air of at least 130 mJ;
a wideband spark ignition energy at about 5.50 vol% of the composition in air of at least 240 mJ;
a wideband spark ignition energy at about 5.25 vol% of the composition in air of at least 450 mJ; and
a wideband spark ignition energy at about 5.00 vol% of the composition in air of at least 670 mJ.

3. The composition of claim 1 or claim 2, further comprising one or more of:
a GWP from 0.06 to 1.0;
a wideband spark ignition energy at about 5.75 vol% of the composition in air of at most 140 mJ;
a wideband spark ignition energy at about 5.50 vol% of the composition in air of at most 270 mJ;
a wideband spark ignition energy at about 5.25 vol% of the composition in air of at least 520 mJ; and
a wideband spark ignition energy at about 5.00 vol% of the composition in air of at least 800 mJ.

4. The composition of any of claims 1-3, wherein the composition comprises at least 99.9 wt% 1,1-difluoroethylene (HFO-1132a), based on a total weight of the composition, and one or more of the following impurities, based on a total weight of the composition:
at most 800 wppm of a combination of difluoromethane (HFC-32), 1,1-difluoroethane (HFC-152a), 1,2-difluoroethane (HFC-152), 1,2-difluoroethylene (HFO-1132, both cis- and trans- isomers), and trifluoroethylene (HFO-1123);
at most 100 wppm of a combination of tetrafluoromethane (HFC-14), trifluoromethane (HFC-23), fluoromethane (HFC-41);
at most 200 wppm of a combination of fluoroethylene (HFO-1141), tetrafluoroethylene (HFO-1114), 3,3,3-trifluoro-1-propene (HFC-1243zf), 2,3,3,3-tetrafluoro-1-propene (HFO-1234yf), 1,3,3,3-tetrafluoro-1-propene (HFO-1234ze), and hexafluoropropene (HFO-1216);
at most 50 wppm of all organic compounds containing a chlorine, bromine, and/or iodine atom;
at most 50 wppm of a combination of fluoroethane (HFC-161), 1,1,1-trifluoroethane (HFC-143a), 1,1,2-trifluoroethane (HFC-143), 1,1,1,2-tetrafluoroethane (HFC-134a), 1,1,2,2-tetrafluoroethane (HFC-134), pentafluoroethane (HFC-125), and hexafluoroethane (HFC-116);
at most 70 wppm of a combination of methane, ethane, ethylene, acetylene, propane, propylene, butane, and isobutane;
at most 50 wppm of a combination of Group 1 metals, Group 2 metals, Group 13 metals, Group 14 metals, Group 15 metals, transition metals, rare earth metals, and metalloids; and
at most 100 wppm of a combination of carbon dioxide, ammonia, hydrogen fluoride, hydrogen chloride, chlorine, and water.

5. The composition of any of claims 1-4, wherein the composition comprises at least 99.99 wt% 1,1-difluoroethylene (HFO-1132a), based on a total weight of the composition, and at most 100 wppm of a combination of difluoromethane (HFC-32), 1,1-difluoroethane (HFC-152a), 1,2-difluoroethane (HFC-152), 1,2-difluoroethylene (HFO-1132, both cis- and trans- isomers), trifluoroethylene (HFO-1123), tetrafluoromethane (HFC-14), trifluoromethane (HFC-23), fluoromethane (HFC-41), fluoroethylene (HFO-1141), tetrafluoroethylene (HFO-1114), 3,3,3-trifluoro-1-propene (HFC-1243zf), 2,3,3,3-tetrafluoro-1-propene (HFO-1234yf), 1,3,3,3-tetrafluoro-1-propene (HFO-1234ze), hexafluoropropene (HFO-1216), fluoroethane (HFC-161), 1,1,1-trifluoroethane (HFC-143a), 1,1,2-trifluoroethane (HFC-143), 1,1,1,2-tetrafluoroethane (HFC-134a), 1,1,2,2-tetrafluoroethane (HFC-134), pentafluoroethane (HFC-125), hexafluoroethane (HFC-116), all organic compounds containing a chlorine, bromine, and/or iodine atom, methane, ethane, ethylene, acetylene, propane, propylene, butane, isobutane, Group 1 metals, Group 2 metals, Group 13 metals, Group 14 metals, Group 15 metals, transition metals, rare earth metals, metalloids, carbon dioxide, ammonia, hydrogen fluoride, hydrogen chloride, chlorine, and water.

6. A method for purifying a relatively impure 1,1-difluoroethylene (HFO-1132a) composition comprising:
providing a relatively impure 1,1-difluoroethylene (HFO-1132a) composition comprising: a first content from 70 wt% to 99.99 wt% of 1,1-difluoroethylene (HFO-1132a), based on a total weight of the composition; a first content of difluoromethane (HFC-32); a first content of 1,2-difluoroethylene (HFO-1132, collectively cis- and trans- isomers); a first content of difluoroethane (HFC-152, collectively both 1,1- and 1,2- isomers); a first content of trifluoromethane (HFC-23); a first content of fluoromethane (HFC-41); a first content, collectively, of all organic compounds containing a chlorine, bromine, and/or iodine atom; a first content of fluoroethylene (HFO-1141); a first content of hexafluoropropene (HFO-1216); a first content of fluoroethane (HFC-161); a first content of trifluoroethane (HFC-143, collectively both 1,1,1- and 1,1,2- isomers); a first content of tetrafluoroethane (HFC-134, collectively 1,1,1,2- and 1,1,2,2- isomers); a first content of pentafluoroethane (HFC-125); a first content of hexafluoroethane (HFC-116); a first combined content of ethylene and propylene; and a first combined content of water;
exposing the relatively impure 1,1-difluoroethylene (HFO-1132a) composition to a purification process under conditions so as to form a relatively pure 1,1-difluoroethylene (HFO-1132a) composition, wherein the relatively pure 1,1-difluoroethylene (HFO-1132a) composition comprises: a second content from 99.9 wt% to 99.9999 wt% of 1,1-difluoroethylene (HFO-1132a), based on a total weight of the relatively pure composition, which second content is at least 0.1 wt% greater than its corresponding first content; a second content of difluoromethane (HFC-32); a second content of 1,2-difluoroethylene (HFO-1132, collectively cis- and trans-isomers); a second content of difluoroethane (HFC-152, collectively both 1,1- and 1,2- isomers); a second content of trifluoromethane (HFC-23); a second content of fluoromethane (HFC-41); a second content, collectively, of all organic compounds containing a chlorine, bromine, and/or iodine atom; a second content of fluoroethylene (HFO-1141); a second content of hexafluoropropene (HFO-1216); a second content of fluoroethane (HFC-161); a second content of trifluoroethane (HFC-143, collectively both 1,1,1- and 1,1,2- isomers); a second content of tetrafluoroethane (HFC-134, collectively 1,1,1,2- and 1,1,2,2- isomers); a second content of pentafluoroethane (HFC-125); a second content of hexafluoroethane (HFC-116); a second combined content of ethylene and propylene; and a second combined content of water, wherein the second content of difluoromethane (HFC-32) and optionally at least one other of said second contents and second combined contents is(are) at least 25% lower than its(their) corresponding first content(s); and
optionally isolating and/or storing said relatively pure 1,1-difluoroethylene (HFO-1132a) composition.

7. The method of claim 6, wherein the relatively pure 1,1-difluoroethylene (HFO-1132a) composition from the purification process comprises no second content or second combined content is more than 30 wppm or more than 200% higher than its corresponding first content or first combined content.

8. The method of claim 6 or claim 7, wherein the purification process comprises a distillation step to capture overhead/gas-phase product, a distillation step to capture bottoms/liquid and/or solid phase product, an azeotropic distillation step, an absorption step, a drying and/or solvent removal step, an extraction step, a filtration step, a membrane separation step, or a combination thereof.

9. The method of any one of claims 6-8, further comprising passing said relatively pure 1,1-difluoroethylene (HFO-1132a) composition to a plasma etching process.

10. The method of claim 9, wherein the plasma etching process comprises:
providing a multilayered substrate comprising one or more trenches, slits, vias, through-holes, staircase contacts, capacitor holes, contact holes, connectors, or other features spanning more than one layer to be selectively etched in an etching chamber;
supplying the relatively pure 1,1-difluoroethylene (HFO-1132a) composition to the etching chamber, immediately prior to or during which supplying step the relatively pure 1,1-difluoroethylene (HFO-1132a) composition is converted into a plasma;
allowing the plasma to selectively etch one or more of the trenches, slits, vias, through-holes, staircase contacts, capacitor holes, contact holes, connectors, or other features spanning more than one layer of the multilayered substrate.

11. The method of claim 10, wherein the plasma also passivates one or more surfaces of the multilayer substrate and/or of one or more of its features.

12. The method of claim 10 or claim 11, wherein the substrate comprises silicon and wherein at least one of the layers comprises silicon nitride, silicon oxide, silicon oxynitride, crystalline Si, polycrystalline silicon, amorphous silicon, polysilicon, silicon oxide hydroxide, silicon carbide, carbon-containing silicon oxide, graphene, amorphous carbon, or other carbonaceous conductive and/or dielectric materials, optionally other metals and/or metalloids in their oxide, nitride, oxynitride, carbide, or other forms, and combinations thereof.

13. The method of any of claims 9-12, wherein generation of the plasma involves adding: an inert gas comprising helium, nitrogen, neon, argon, krypton, xenon, or a combination thereof; a reactive gas comprising hydrogen and/or a source of hydrogen, carbon monoxide, carbon dioxide, carbonyl sulfide, nitric oxide, nitrogen oxide, nitrous oxide, oxygen, ozone, hydrogen sulfide, sulfur dioxide, or a combination thereof; or both.

14. The method of any of claims 6-13, wherein the etching conditions include imposition of cryogenic temperatures ranging from -5°C to -80°C in or on a wall of the etching chamber.

15. The method of any of claims 6-14, wherein the plasma is generated and/or maintained using an energy from 5 eV to 75 eV
